# EUROPÄISCHE PATENTSCHRIFT

(11) **EP 3 730 220 B1**
(45) Veröffentlichungstag und Bekanntmachung des Hinweises auf die Patenterteilung: **06.07.2022**
(21) Anmeldenummer: 19171385.8
(22) Anmeldetag: 26.04.2019
(51) Int. Cl.: B05B 11/00

(54) **SPENDER ZUM AUSTRAG PHARMAZEUTISCHER FLÜSSIGKEITEN**
DISPENSER FOR DISCHARGING LIQUIDS
DISTRIBUTEUR DESTINÉ À LA SORTIE DES LIQUIDES PHARMACEUTIQUES

(43) Veröffentlichungstag der Anmeldung: 28.10.2020
(73) Patentinhaber: Aptar Radolfzell GmbH, 78315 Radolfzell (DE)
(72) Erfinder: Greiner-Perth, Jürgen, 78244 Gottmadingen (DE); Krampen, Gerald, 78315 Radolfzell (DE)
(74) Vertreter: Witte, Weller und Partner Patentanwälte mbB Stuttgart

(56) Entgegenhaltungen:
- EP-A1- 0 784 513
- WO-A1-2004/062422
- WO-A1-2013/057223
- DE-A1- 1 907 358
- DE-A1- 4 141 825
- DE-B- 1 491 706
- DE-T2- 68 903 116
- FR-A- 1 531 558
- FR-A1- 2 877 325
- US-A- 2 884 164
- US-A- 5 454 488

## Beschreibung

Die Erfindung betrifft einen Spender zum Austrag pharmazeutischer Flüssigkeiten, insbesondere für den tropfenweisen Austrag von pharmazeutischen Flüssigkeiten in Form von Einzeltropfen. Es kann sich beispielsweise um Augentropfen, Ohrentropfen oder Nasentropfen handeln.

Ein gattungsgemäßer Spender weist einen Flüssigkeitsspeicher, eine Pumpeinrichtung mit einer volumetrisch veränderlichen Pumpkammer sowie eine Abgabeöffnung zur Abgabe der Flüssigkeit auf. Zur Veränderung des Volumens der Pumpkammer zum Zwecke des Ansaugens von Flüssigkeit aus dem Flüssigkeitsspeicher sowie zum Druckbeaufschlagen der Flüssigkeit zum Zwecke des Austrags ist die Pumpkammer an einer Seite durch ein Membranteil begrenzt, das zumindest teilweise aus einem weichen Kunststoff besteht und somit bei gleichzeitiger Verformung verlagerbar ist. Ein gattungsgemäßer Spender weist weiterhin ein Gehäuse auf, innerhalb dessen die Pumpeinrichtung vorgesehen ist. Ein gegenüber dem Gehäuse zwischen einer unbetätigten Ausgangslage und einer betätigten Endlage verlagerbarer Betätigungstaster ist zur Verlagerung/Verformung des Membranteils zum Zwecke des Flüssigkeitsaustrags vorgesehen.

Aus der DE 102006012898 A1, Ausgestaltung der Fig. 2a und 3b, ist bereits ein Tropfenspender mit einer Pumpeinrichtung bekannt, deren Pumpkammer auf einer Seite durch ein Membranteil begrenzt ist. Einige Aspekte des dort beschriebenen Spenders sind jedoch nachteilig. So sind insbesondere die Handhabung des weichen Membranteils vor und bei der Montage sowie die Kopplung des Betätigungstasters und des Membranteils miteinander während der Montage in der Umsetzung der automatisierten Montage problematisch. Weiterhin bereiten die aus dem Stand der Technik bekannten Gestaltungen Probleme bei der Inbetriebnahme, da die Anordnung und Ausgestaltung der Ventile und derVolumina der Kanäle und der Pumpkammer ein initiales Austreiben der Luft erschweren. Aus der DE1491706 B1 ist ein Zerstäuber für flüssige pharmazeutische Produkte bekannt.

### AUFGABE UND LÖSUNG

Aufgabe der Erfindung ist es, einen beschriebenen gattungsgemäßen Spender mit einer durch ein Membranteil begrenzten Pumpkammer dahingehend weiterzubilden, dass die beschriebenen Nachteile überwunden oder zumindest verringert werden.

Zur Lösung der Aufgabe wird ein Spender vorgeschlagen, der einen Flüssigkeitsspeicher zur Lagerung der Flüssigkeit vor dem Austrag, ein Gehäuse mit einer darin integrierten Pumpeinrichtung mit einer volumetrisch veränderlichen Pumpkammer sowie eine Abgabeöffnung zur Abgabe der Flüssigkeit aufweist. Die Pumpeinrichtung dient dem Zweck, Flüssigkeit aus dem Flüssigkeitsspeicher anzusaugen und bei einer Betätigung des Spenders durch die Abgabeöffnung abzugeben.

Die Pumpkammer ist an einer Seite durch das in sich zumindest abschnittsweise verformbare Membranteil, welches zumindest im Bereich einer Pumpkammerwandung aus einem weichen und somit leicht verformbaren Kunststoff besteht, verschlossen. Andere Wandungsteile der Pumpkammer werden durch starre Gehäuseabschnitte gebildet, die dicht mit dem Membranteil verbunden sind. Das Membranteil kann in Richtung der Pumpkammer eingedrückt werden, um das Volumen der Pumpkammer zu verkleinern und somit Flüssigkeit in Richtung der Abgabeöffnung zu drücken. Üblicherweise ist die Pumpkammer durch zwei Ventile begrenzt, nämlich eingangsseitig durch ein Einlassventil, welches bei einem Unterdruck in der Pumpkammer gegenüber dem Flüssigkeitsspeicher öffnet, sowie ausgangsseitig durch ein Auslassventil, welches bei einem Überdruck in der Pumpkammer gegenüber einem zur Abgabeöffnung führenden Abgabekanal öffnet. Das Einlassventil ist bei identischem Druck im Flüssigkeitsspeicher und in der Pumpkammer geschlossen und öffnet vorzugsweise ab einem Unterdruck von etwa 0,1 bis 0,2 bar in der Pumpkammer. Das Auslassventil ist bei identischem Druck in der Pumpkammer und dem sich anschließenden Abgabekanal geschlossen und öffnet vorzugsweise ab einem Überdruck von etwa 0,5 bar in der Pumpkammer. Vorzugsweise ist der Abgabeöffnung ein weiteres Ventil vorgeschaltet, welches bei einem Überdruck der Flüssigkeit im Abgabekanal gegenüber einem Umgebungsdruck öffnet. Dieses Abgabeventil ist vorzugsweise dafür ausgebildet, bei einem Überdruck von mindestens 0,3 bar, vorzugsweise mindestens 0,5 bar, zu öffnen. Der Überdruck zum Öffnen des Ventils sollte bei pharmazeutischen Flüssigkeiten ohne Konservierungsmittel nicht zu gering sein, um möglichst zu verhindern, dass externe Druckschwankungen ein Öffnen bewirken. Bei Flüssigkeiten mit Konservierungsmittel sind die Anforderungen geringer. Hier können auch Öffnungsdrücke des Abgabeventils hinab bis 0,05 bar möglich und vorteilhaft sein.

Der Spender weist zur Betätigung der Pumpeinrichtung einen zwischen einer unbetätigten Ausgangslage und einer betätigten Endlage verlagerbaren Betätigungstaster auf, dessen Verlagerung in die Endlage zur Verlagerung/Verformung des Membranteils und damit zur Verkleinerung der Pumpkammer zum Zwecke des Flüssigkeitsaustrags führt.

Wenngleich es für die nachfolgend beschriebenen Aspekte der Erfindung nicht grundsätzlich zwingend ist, wird die Realisierung der beschriebenen Maßnahmen insbesondere bei einem so genannten Side-Actuation-Spender als vorteilhaft angesehen. Bei einem solchen Side-Actuation-Spender weist das Gehäuse des Spenders eine in Richtung einer Haupterstreckungsrichtung ausgerichtete längliche Außenform auf, wobei am distalen Ende des Spenders die Abgabeöffnung vorgesehen ist. Das Gehäuse kann einen im Wesentlichen runden Querschnitt aufweisen, kann aber auch hiervon abweichen und eine eher flache Bauform aufweisen. Der Betätigungstaster ist seitlich am Gehäuse dieses Spenders vorgesehen und in Richtung einer Mittelachse eindrückbar, um hierdurch das Membranteil zur Verkleinerung der Pumpkammer zum Zwecke des Flüssigkeitsaustrags zu verformen. Die Betätigung des Betätigungstasters erfolgt dabei üblicherweise mittels des Daumens.

Die nachfolgend beschriebenen Aspekte der Erfindung sind insbesondere bei der Realisierung eines Tropfenspenders von Vorteil, also eines Spenders, mit dem bestimmungsgemäß pharmazeutische Flüssigkeit in Form einzelner Tropfen abgegeben wird. Hierfür ist vorzugsweise jenseits der Abgabeöffnung ein Tropfenbildungsmittel vorgesehen, also ein Bereich, in dem die abgegebene Flüssigkeit sich sammelt, bis ihr Volumen reicht, um einen Tropfen zu bilden, der sich schwerkraftbedingt von den Tropfenbildungsmitteln löst. Insbesondere kann es sich bei den Tropfenbildungsmitteln um eine die Abgabeöffnung umgebende Tropfenbildungsfläche handeln, die insbesondere eben oder konkav geformt und/oder außenseitig durch eine scharfkantige Abrisskante mit einem Krümmungsradius von vorzugsweise weniger als 0,5mm begrenzt ist.

Sofern ein erfindungsgemäßer Spender als Tropfenspender ausgestaltet ist, weist die Pumpkammer vorzugsweise ein nutzbares Volumen, gebildet durch die Volumendifferenz zwischen Maximalvolumen und Minimalvolumen der Pumpkammer, auf, welches zwischen 20 µl und 50 µl, vorzugsweise zwischen 30 µl und 40 µl, beträgt. Ein solches Volumen entspricht in etwa dem Volumen eines Tropfens bei üblichen Gestaltungen von Tropfenbildungsmitteln. Der Benutzer kann somit durch eine Betätigung des Betätigungstasters genau einen Tropfen ausbringen. Sofern die Tropfenbildungsmittel zur Bildung von größeren Tropfen ausgebildet sind, ist das nutzbare Volumen der Pumpkammer vorzugsweise entsprechend größer. Auch bei der Gestaltung des Spenders als Tropfenspender kann eine größere Pumpkammer zweckmäßig sein, um mit einer Betätigung mehrere Tropfen abgeben zu können.

Um trotz eines vergleichsweise geringen Pumpkammervolumens die bei Inbetriebnahme in der Pumpkammer befindliche Luft aus dieser hinausdrücken zu können, ist ein Auslassventil der Pumpkammer vorzugsweise dafür ausgebildet, bereits bei nur geringem Überdruck zu öffnen. Insbesondere jedoch ist es von Vorteil für eine problemlose Inbetriebnahme des Spenders, wenn der Quotient zwischen einem minimalen Pumpkammervolumen und einem maximalen Pumpkammervolumen maximal 1:2 beträgt, vorzugsweise maximal 1:3. Ein solcher Quotient lässt sich insbesondere mit den nachfolgend erläuterten erfindungsgemäßen Maßnahmen erzielen.

Gemäß einem ersten Aspekt der Erfindung weist der genannte Betätigungstaster ein Tasterbauteil aus einem starren Kunststoff auf. Weiterhin weist der Spender eine Federeinrichtung auf, durch die der Betätigungstaster in Richtung seiner unbetätigten Ausgangslage kraftbeaufschlagt ist, so dass er nach einem Niederdrücken des Betätigungstasters zurück in die Ausgangslage gedrückt wird.

Dabei ist das Membranteil, das die Pumpkammer begrenzt, mit dem genannten Tasterbauteil des Betätigungstasters verbunden, so dass der Betätigungstaster bei der durch die Federeinrichtung bewirkten Verlagerung in seiner Ausgangslage das Membranteil mitzieht. Das Membranteil kann somit aus einem Kunststoff gefertigt sein, der selbst keine starke inhärente Rückstellneigung aufweist, sondern mittelbar von der Rückstellfeder zurückgezogen wird. Die Verbindung zwischen dem weichen und verformbaren Kunststoff des Membranbauteils und dem demgegenüber härteren und starreren Kunststoff des Tasterbauteils ist dadurch realisiert, dass ein Verbindungabschnitt des Membranteils zwischen mindestens zwei Klemmflächen des Tasterbauteils eingeklemmt ist.

Es hat sich gezeigt, dass die Technik des Einklemmens einerseits gut geeignet ist, um Zugkräfte vom Tasterbauteil auf das Membranteil zu übertragen und andererseits vergleichsweise gut in einer automatisierten Montage realisierbar ist. Die beiden Klemmflächen können hierzu während der Montage beispielsweise durch eine Art Spreizer auseinandergedrückt werden, so dass ein Abschnitt des Membranbauteils zwischen die mindestens zwei Klemmflächen eingeführt werden kann und anschließend der Spreizer herausgezogen wird. Die Klemmflächen legen sich dann beidseitig an den eingeschobenen Abschnitt des Membranteils an, wobei auf den Umfang des Verbindungsabschnitts bezogen zwischen den Klemmflächen jeweils Bereiche verbleiben, in denen das Tasterbauteil sich nicht in Kontakt mit dem Verbindungsabschnitt befindet.

Obwohl eine Anzahl von zwei Klemmflächen als üblicherweise ausreichend angesehen wird, können auch mehr Klemmflächen Verwendung finden. Beispielsweise sind auch drei oder mehr Klemmflächen, die sternartig aufeinander zu ragen, eine zweckmäßige Bauform.

Die Klemmflächen können eben ausgebildet sein. Um die übertragbaren Zugkräfte noch zu erhöhen, kann es aber auch sinnvoll sein, sie jeweils mit einer scharfkantigen Eindringgeometrie mit einem Krümmungsradius kleiner 0,3 mm an der scharfen Kante zu versehen, die entsprechend tiefer in den eingeschobenen Abschnitt des Membranteils eindringen kann und die Klemmwirkung somit erhöht.

Bei einer bevorzugten Ausgestaltung weist das Tasterbauteil eine als längliche Durchbrechung ausgebildete Kopplungsdurchbrechung auf, an deren Rand einander gegenüberliegend die beiden Klemmflächen vorgesehen sind. Eine solche längliche Durchbrechung ist in Richtung ihrer länglichen Erstreckung dann vorzugsweise um mindestens Faktor 2 oder 3 größer als der Abstand zwischen den aufeinander zu weisenden Klemmflächen. Die längliche Erstreckung erleichtert die Aufweitung bei der Montage zum Zwecke der Einfügung des Membranteils.

Insbesondere von Vorteil ist es weiterhin, wenn parallel zur Kopplungsdurchbrechung mindestens eine als längliche Durchbrechung ausgebildete Montagedurchbrechung im Tasterbauteil vorgesehen ist. Vorzugsweise ist beidseitig der Kopplungsdurchbrechung jeweils eine solche Montagedurchbrechung vorgesehen. Die mindestens eine Kopplungsdurchbrechung ermöglicht während der Montage ein Auslenken mindestens einer Klemmfläche zum Zwecke des Einfügens des Verbindungsabschnitts des Membranteils, ohne dass der Kunststoff des Tasterbauteils hierfür stark verformt werden muss und Schaden nehmen kann.

Gemäß einem zweiten Aspekt der Erfindung ist vorgesehen, dass der Spender auf einer dem Membranteil gegenüberliegenden Seite der Pumpkammer eine Gegenwandung aufweist. Diese ist vorzugsweise aus einem starren Bauteil gebildet. In diese Gegenwandung ist ein Ventilbauteil eingesetzt. Dieses Ventilbauteil weist mindestens eine Ventilfläche eines Einlassventils oder eines Auslassventils der Pumpeinrichtung auf, wobei diese Ventilfläche vorzugsweise zur Anlage an einer Oberfläche des Gehäusebauteils der Gegenwandung im geschlossenen Zustand des Ventils ausgebildet ist. Insbesondere von Vorteil ist es, wenn das Ventilbauteil Ventilflächen sowohl des Einlassventils als auch des Auslassventils aufweist, wobei hierbei eine Bauweise bevorzugt wird, bei der ein Einlasskanal in die Pumpkammer und ein Auslasskanal in die Pumpkammer konzentrisch zueinander angeordnet sind, so dass ein einziges Ventilbauteil an einer Wandung zwischen dem Einlasskanal und dem Auslasskanal angeordnet ist und sowohl eine nach innen weisende als auch eine nach außen weisende Ventillippe aufweist.

Das Membranteil ist gemäß diesem Aspekt der Erfindung im Zuge der Betätigung derart verlagerbar, dass es in einer betätigten Endlage zumindest abschnittsweise an diesem Ventilbauteil anliegt, insbesondere zumindest abschnittsweise an einer Ventilfläche in Form einer Ventillippe aus elastischem Kunststoff anliegt.

Eine solche Verlagerbarkeit des Membranteils bis zum gegenüberliegenden Ventilbauteil ermöglicht ein sehr geringes Minimalvolumen der Pumpkammer. In seiner Endlage verringert das Membranteil den Abstand zwischen Membranteil und Ventilbauteil zumindest abschnittsweise auf Null. Insbesondere von Vorteil ist es, wenn Flächen aus leicht verformbarem Kunststoff des Membranteils und des Ventilbauteils aneinander zu Anlage kommen, da sich diese besonders gut aneinander anpassen und somit die Verringerung des Minimalvolumens weiter begünstigen. In einer besonderen Ausgestaltung kann zudem vorgesehen sein, dass das Membranteil in seiner Endlage derart auf das Ventilbauteil drückt, dass das Einlassventil oder das Auslassventil mechanisch zwangsgeöffnet wird, um in der Pumpkammer vorhandene komprimierte Luft entweichen zu lassen.

Das Membranteil weist vorzugsweise einen in die Pumpkammer hineinragenden Mittelteil auf, der eine an die Form des Ventilbauteils angepasste Form aufweist, wobei vorzugsweise der Mittelteil eine in Richtung des Ventilbauteils sich verjüngende Formgebung aufweist und das Ventilbauteil eine durch eine Einlassventillippe bedingte komplementäre kelchartige Formgebung aufweist, in die das Mittelteil einrückt. Auch dies begünstigt die Reduzierung des Minimalvolumens der Pumpkammer.

Weiterhin wird es als bevorzugt angesehen, wenn das Membranteil eine den Mittelteil des Membranteils umgebende Ringfläche aufweist. Diese Ringfläche wird bei einer Verformung des Membranteils während der Verkleinerung der Pumpkammer quasi umgestülpt. Ein Ringbereich dieser Ringfläche, der im Ausgangszustand nach außen weist, weist dadurch in der durch Betätigung bewirkten Endlage des Membranteils nach innen. Dieses Umstülpen oder Abrollen ermöglicht es, das minimale Pumpkammervolumen gering zu halten, da die durch das Membranteil gebildete Pumpkammerwandung sich im abgerollten Zustand bündig an eine starre Mantelwandung der Pumpkammer anlegen kann.

Erfindungsgemäß ist vorgesehen, dass das Membranteil in einem äußeren Randbereich umlaufend an einem Gehäuseabschnitt des Gehäuses befestigt ist. Dabei ist zur Befestigung des Randbereichs des Membranteils am Gehäuseabschnitt ein Befestigungsring aus einem starren Kunststoff vorgesehen, wobei unter einem starren Kunststoff in diesem Zusammenhang ein Kunststoff verstanden wird, dessen E-Modul größer ist als das E-Modul des Membranteils in dem Bereich der Pumpkammerwandung, die bei einer Verkleinerung der Pumpkammer verformt wird.

Nicht erfindungsgemäß kann der Befestigungsring gegenüber dem Membranteil als getrenntes Bauteil ausgebildet sein, welches erst während der Montage in Kontakt mit dem Membranteil gelangt und diesen abdichtend gegen den genannten Gehäuseabschnitt presst oder selbst angepresst und abdichtend am Gehäuseabschnitt anliegt.

Erfindungsgemäß ist allerdings eine einstückige Gestaltung, bei der das Membranteil aus unterschiedlichen Kunststoffen besteht, die durch Mehrkomponentenspritzguss unmittelbar zusammengefügt sind. Der genannte weichere Kunststoff bildet dabei das Material für die bestimmungsgemäß während der Betätigungsich verformenden Teile, insbesondere für den bereits genannten Ringbereich des Membranteils. Der demgegenüber starrere Bauteil bildet einen hieran angeformten umlaufenden Bereich, der somit den genannten Befestigungsring bildet.

Im montierten Zustand sorgt der Befestigungsring dafür, dass der Rand des Membranteils sicher am Gehäuseabschnitt fixiert ist. Dies ist insbesondere dann vorteilhaft, wenn besonders weicher Kunststoff für die im Betrieb bestimmungsgemäß zu verformenden Abschnitte des Membranteils gewählt wird, da ein solches Material ohne zusätzlichen Befestigungsring sich nicht gut für die Fixierung am Gehäuse eignet.

Wenn der Befestigungsring erfindungsgemäß einstückigerTeil des Membranteils ist, wird hierdurch zudem die Handhabung dieses Bauteils während der Montage erheblich vereinfacht. Der durch den Befestigungsring gebildete starre Teil des Membranteils erleichtert ausgehend von den ungerichtet zugeführten Membranteilen die Vereinzelung und Ausrichtung für die nachfolgende Montage.

Der Gehäuseabschnitt, an dem das Membranteil mittels des Befestigungsrings fixiert wird, ist vorzugsweise ein ringförmiger Gehäuseabschnitt, wobei der Befestigungsring an einer Außenseite dieses ringförmigen Gehäuseabschnitts aufgeklemmt ist und wobei eine Innenseite des Gehäuseabschnitts eine umlaufende Wandung der Pumpkammer bildet.

Gemäß einem dritten Aspekt der Erfindung ist der Betätigungstaster als Kipptaster ausgebildet, der um eine Kippachse kippbeweglich gegenüber dem Gehäuse ausgebildet ist. Der Kipptaster weist dabei ein starres Tasterbauteil auf, welches bei der Schwenkbewegung des Betätigungstasters auf das Membranteil drückt und dadurch die Pumpkammer verkleinert.

Die Verwendung eines Kipptasters gestattet eine recht genaue Dosierung. Dies hängt vor allem damit zusammen, dass der Kipptaster durch seine schwenkbare Anlenkung nicht zum Verkanten oder dergleichen neigt und dass der Benutzer durch den Abstand der manuellen Kraftbeaufschlagung von der Kippachse gut steuern kann, mit welcher Geschwindigkeit der Kipptaster eingedrückt wird. Dies kann insbesondere bei der Tropfenabgabe von Vorteil sein, da hierdurch der Ablösezeitpunkt des Tropfens von den Tropfenbildungsmitteln genau beeinflussbar ist.

Die Verwendung eins Kipptasters ist insbesondere im Kontext des erfindungsgemäß vorgesehenen Membranteils zweckmäßig, da das Membranteil durch seine Verformbarkeit gut geeignet ist, um eine Schwenkbewegung eines Kipptasters in eine im Wesentlichen lineare Bewegung der verlagerbaren Pumpkammerwandung zu überführen. Auch ist es fertigungstechnisch sehr einfach, das Membranteil mit einem elastisch verformbaren Ausgleichsabschnitt auszubilden, der zwischen der Pumpkammerwandung und dem Kipptaster angeordnet ist.

Die Kippachse des Kipptasters ist vorzugsweise bezogen auf die Haupterstreckungsrichtung an einem von der Abgabeöffnung weg weisenden Ende des Kipptasters vorgesehen. Die Kippachse ist vorzugsweise orthogonal zur Haupterstreckungsachse des Spenders ausgerichtet. Grundsätzlich kann eine solche Schwenkbeweglichkeit mittels eines einfachen Filmscharniers erzielt werden. Demgegenüber bevorzugt ist es jedoch, wenn am Kipptaster oder an einem umgebenden Gehäuseabschnitt Achsabschnitte vorgesehen sind, die in Lager auf der jeweils anderen Seite eingreifen und hierdurch die Kippbeweglichkeit ermöglichen.

Wie oben schon erwähnt, weist der Spender vorzugsweise eine Federeinrichtung auf, die auf den Betätigungstaster wirkt und durch die der Betätigungstaster in Richtung seiner unbetätigten Ausgangslage kraftbeaufschlagt wird. Diese Federeinrichtung kann grundsätzlich als einstückig mit dem Betätigungstaster und/oder einstückig mit dem Gehäuse ausgebildeter elastisch verformbarer Federabschnitt ausgebildet sein. Bevorzugt wird allerdings eine separate Federeinrichtung, insbesondere in Form einer Schraubenfeder aus Kunststoff oder Metall. Die Federeinrichtung drückt den Betätigungstaster in seiner unbetätigten Ausgangslage, wobei hierbei die Pumpkammer wieder vergrößert wird und dabei Flüssigkeit aus dem Flüssigkeitsspeicher ansaugt.

Grundsätzlich möglich ist eine Bauweise, bei der die Federeinrichtung einerseits und die Verbindung des Kipptasters zum Membranteil andererseits identisch von der Kippachse beabstandet sind. Dies ergibt sich beispielsweise, wenn die Federeinrichtung als Schraubenfeder ausgestaltet ist, die konzentrisch zur Anbringung des Membranteils am Kipptaster das Membranteil umgebend angeordnet ist.

Als vorteilhaft wird es jedoch angesehen, wenn ein Verbindungsbereich, mit dem der Kipptaster auf das Membranteil drückt, und ein Krafteinkopplungsbereich, in dem die Federeinrichtung eine Rückstellkraft in den Kipptaster einkoppelt, unterschiedlich weit von der Kippachse entfernt am Kipptaster vorgesehen sind. Dabei wird es also insbesondere vorteilhaft angesehen, wenn der Krafteinkopplungsbereich weiter von der Kippachse entfernt ist als der Verbindungsbereich. Weiterhin wird es als vorteilhaft angesehen, wenn die Abstände zur Kippachse sich mindestens um 20% voneinander unterscheiden und somit signifikant unterschiedlich sind. Voneinander abweichende Abstände des Verbindungsbereichs und des Krafteinkopplungsbereichs zur Kippachse weisen verschiedene Vorteile auf, insbesondere auch die einfachere Montage, da die Rückstellfeder und die Verbindung mit der verlagerbaren Pumpkammerwandung örtlich voneinander getrennt sind.

Vorzugsweise weist ein erfindungsgemäßer Spender eine abnehmbare Spenderkappe auf, die auf das Gehäuse des Spenders aufsetzbar ist und von diesem zur Nutzung des Spenders abgenommen wird. Diese Spenderkappe schützt im aufgesetzten Zustand die Abgabeöffnung. Insbesondere vorzugweise ist eine solche Spenderkappe als belüftete Spenderkappe ausgestaltet und weist hierfür mindestens eine Ventilationsöffnung auf, durch die die Abgabeöffnung auch bei aufgesetzter Spenderkappe mit einer umgebenden Atmosphäre verbunden ist. Dies begünstigt ein schnelles Trocknen eines dort verbliebenen Resttropfens. Im Lieferzustand ist die Ventilationsöffnung vorzugsweise verschlossen und wird vom Benutzer im Zuge der Erstbenutzung geöffnet. Die Spenderkappe verfügt vorzugsweise über einen Sterilfilter, der die Ventilationsöffnungen überdeckt, um Keimeintrag zu verhindern. Weiterhin weist die Spenderkappe vorzugsweise ein integriertes Pad auf, welches bei aufgesetzter Spenderkappe derart über oder an der Abgabeöffnung positioniert ist, dass ein stromabwärts der Abgabeöffnung verbleibender Flüssigkeitsrest hiervon aufgenommen und/oder dekontaminiert wird.

Der Flüssigkeitsspeicher eines erfindungsgemäßen Spenders ist vorzugsweise an dem der Abgabeöffnung gegenüberliegenden Ende des Spenders vorgesehen. Er kann durch einen separaten Flaschenkörper gebildet sein, der an dem die Pumpeinrichtung umgebenden Gehäuse angekoppelt ist, beispielsweise angeschraubt ist. Bei einer hiervon abweichenden Gestaltung ist eine Mantelwandung des Flüssigkeitsspeichers einstückig mit dem Gehäuse verbunden, wobei vorzugsweise ein Boden des Flaschenkörpers durch ein separates Teil gebildet wird, welches an der Mantelwandung befestigt ist. Sofern der Flüssigkeitsspeicher ein unveränderliches Volumen aufweist, verfügt der Spender vorzugsweise über einen Belüftungskanal, insbesondere mit Sterilfilter, durch den der Flüssigkeitsspeicher mit einer umgebenden Atmosphäre verbunden ist. Alternativ dazu kann der Flüssigkeitsspeicher jedoch auch ein variables Innenvolumen aufweisen, insbesondere indem ein Beutel vorgesehen ist, in dem die Flüssigkeit gelagert ist und der von einem starren Flaschenkörper umgeben ist. Auch ein Schleppkolbensystem gestattet die Realisierung eines variablen Innenvolumens.

Ein erfindungsgemäßer Spender ist zur Verwendung für den Austrag pharmazeutischer Flüssigkeiten vorgesehen. Daher ist in einem Lieferzustand der Flüssigkeitsspeicher vorzugweise mit einer solchen pharmazeutischen Flüssigkeit befüllt.

Insbesondere handelt es sich um pharmazeutische Flüssigkeiten zur Behandlung des erhöhten Augeninnendrucks (Glaukombehandlung), zur Behandlung des trockenen Auges und zur Behandlung von Allergien und Entzündungen. Hierbei spielen insbesondere die Molekülgruppen Alpha-2 Agonisten, z. B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z. B. Methylcelluloseverbindungen und Cyclosporin beziehungsweise Antihistaminika, z. B. Olopatadin und Levocabastin, Steroide, z. B. Loteprdnol und Dexamethason, sowie NSAID, z. B. Keterolac, eine Rolle.

Weiterhin ist der erfindungsgemäße Spender vorteilhaft verwendbar für Flüssigkeiten mit Molekülen von einer oder von mehreren der folgenden Sorten : Trichloressigsäure, Trioxysalen, Harnstoff, Zinkoxid, Tacrolimus, Clobetasolpropionat, Mometasonfuroat, Betamethasondipropionat, Fluocinonid, Desoximetasone, Triamcinolon Acetonid, Fluticasonpropionat, Hydrokortison, Clotrimazol, Ketoconazol, Miconazol, Undecylensäure, Terbinafine, Cyclopirox, Tolnaftate, Akziklovir, Imiquimod, Docosanol, Finasterid, Minoxidil, Dexamethason, Tramazolin, Naphazolin, Nostrilla, Oxymethazolin, Phenylephrin, Phenylpropanolamin, Pseudoephedrin, Tetryzolin, Tramazolinhydrochlorid, Tuaminoheptane und Xylometazolin.

Die Komponenten eines erfindungsgemäßen Spenders sind vorzugsweise aus Kunststoff gefertigt. Lediglich für die die beschriebenen Federn und ggf. vorhandene Federn am Einlassventil und/oder dem Auslassventil stellt eine metallische Ausgestaltung eine Alternative dar. Die im Betrieb bestimmungsgemäß verformbaren Teile, insbesondere also der überwiegende Teil des Membranbauteils, die Ventillippen und der Abgabeventilkörper bestehen vorzugsweise aus einem Elastomer-Kunststoff mit einem E-Modul < 200 N/mm². Es kommt beispielsweise ein LDPE (Low Density Polyethylen) in Frage. Die im Betrieb unbeweglichen Teile bestehen vorzugsweise aus einem Kunststoff mit einem E-Modul > 500 N/mm². Hierbei kann es sich beispielsweise um ein HDPE (High Density Polyethylen) oder ein PP (Polypropylen) handeln.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A und 1B zeigen einen erfindungsgemäßen Spender, umfassend eine Haupteinheit sowie Spenderkappe in einer ungeschnittenen Darstellung.
Fig. 2A und 2B zeigen den Spender in einer geschnittenen Darstellung mitsamt der innenliegenden Pumpkammer, die durch ein Membranteil und ein Ventilbauteil begrenzt wird.
Fig. 3 und 4 zeigen den Spender in perspektivischen geschnittenen Darstellungen.
Fig. 5 und 6 zeigen den Betätigungsvorgang des Spenders.
Anhand von Fig. 7 wird die Montagereihe der Einzelteile des Spenders verdeutlicht.
Fig. 8 zeigt die Kopplung zwischen einem dem Betätigungstaster des Spenders und dem Membranteil.
Fig. 9 und 10 zeigen in Details das Ventilbauteil sowie das Membranteil.
Fig. 11 zeigt eine alternative Bauweise des Spenders.

### DETAILLIERTE BESCHREIBUNG DER AUSFÜHRUNGSBEISPIELE

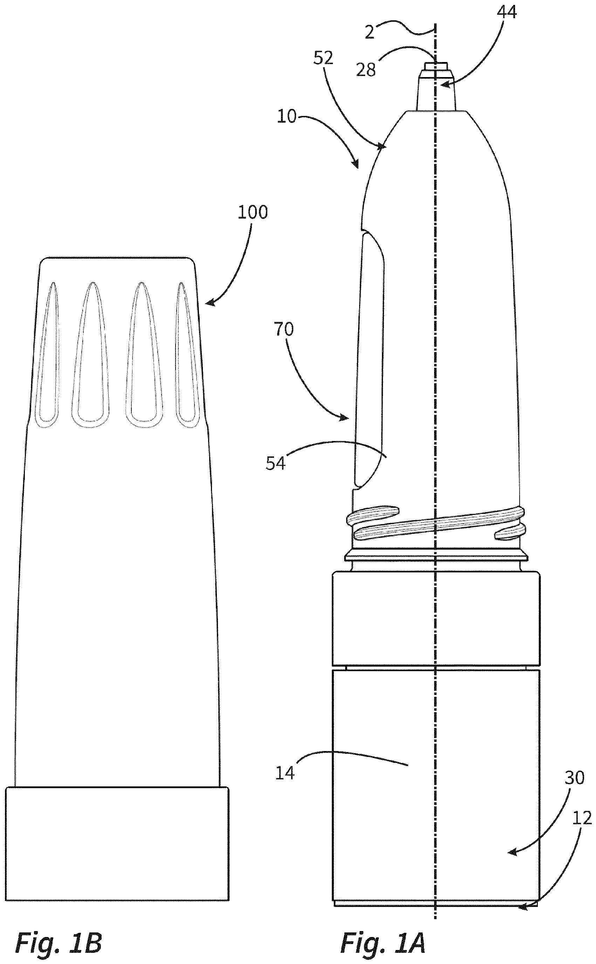

Die Fig. 1A und 1B zeigen ein erstes Ausführungsbeispiel des erfindungsgemäßen Spenders in einer nicht geschnittenen Darstellung mitsamt seiner Spenderkappe. Die in Fig. 1A dargestellte Haupteinheit dieser Ausgestaltung des Spenders weist ein längliches Gehäuse auf, welches aus verschiedenen Gehäusebestandteilen 30, 12, 52, 44 zusammengesetzt ist, die entlang einer Haupterstreckungsrichtung 2 ausgerichtet sind. Wie im Weiteren noch beschrieben ist, ist innerhalb des Gehäuses eine Pumpeinrichtung 16 vorgesehen, mittels der Flüssigkeit aus einem Flüssigkeitsspeicher 14 an einem proximalen Ende des Spenders 10 zu einer Abgabeöffnung 28 an einem distalen Ende des Spenders 10 gefördert werden kann. Zur Betätigung der Pumpeinrichtung 16 ist ein Betätigungstaster 70 vorgesehen.

Die in Fig. 1B dargestellte Spenderkappe 100 kann mittels eines Gewindes auf das Gehäuse des Spenders 10 aufgesetzt werden.

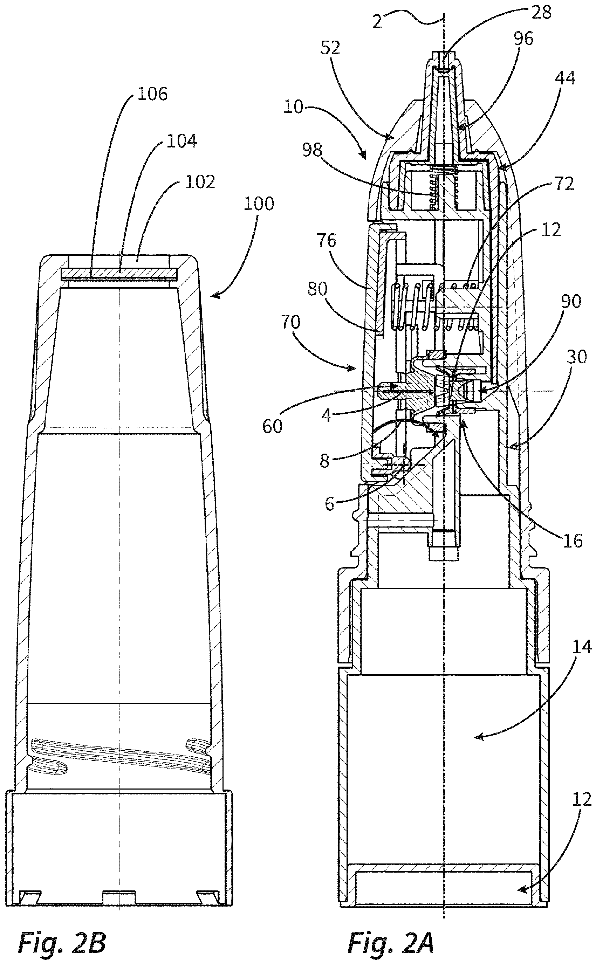

Die Fig. 2A und 2B zeigen die Haupteinheit des Spenders 10 sowie die Spenderkappe 100 in geschnittener Darstellung.

Anhand dieser Darstellung sollen zunächst die Hauptelemente beschrieben werden.

Wie in Fig. 2A ersichtlich ist, besteht die Haupteinheit des Spenders 10 aus mehreren Gehäuseteilen, von denen das erste Gehäusebauteil 30 das größte Gehäusebauteil ist und gleichsam einen zentralen Träger für weitere Bauteile des Spenders darstellt. Dieses Gehäusebauteil 30 stellt an seinem proximalen Ende die Mantelwandung des Flüssigkeitsspeichers 14 zur Verfügung. Es ist Träger verschiedener Bestandteile der Pumpeinrichtung, insbesondere eines Membranteils 60 und eines Ventilbauteils 90, die auf gegenüberliegenden Seiten einer Pumpkammer 20 angeordnet sind. Der Betätigungstaster 70 ist um eine Kippachse 6 schwenkbeweglich am ersten Gehäusebauteil 30 angelenkt. Der Betätigungstaster 70 selbst besteht aus zwei Tasterbauteilen 76, 80, wobei das innenliegende Tasterbauteil 80 in im Weiteren noch beschriebener Art und Weise mit dem Membranteil 60 verbunden ist, so dass das Membranteil 60 mittels des Betätigungstasters 70 in die Pumpkammer 20 eindrückbar ist. Zwischen dem Tasterbauteil 80 und dem ersten Gehäusebauteil ist eine Rückstellfeder 72 vorgesehen.

Am distalen Ende des ersten Gehäusebauteils 30 schließt sich ein zweites Gehäusebauteil 44 an. Dieses zweite Gehäusebauteil 44 ist ortsfest am ersten Gehäusebauteil 30 vorgesehen und dichtet mit diesem gemeinsam einen Innenraum ab, innerhalb dessen ein verformbarer Abgabeventilkörper 96 angeordnet ist, dem seinerseits eine Abgabeventilfeder 98 zugeordnet ist. Ein weiteres Gehäusebauteil, das Außengehäusebauteil 52, umgibt die genannten Gehäusebauteile 30, 44, wobei das Gehäusebauteil 44 mit einer Applikatorspitze 46 durch eine Durchbrechung 58 des Außengehäusebauteils 52 hindurchragt und wobei das Außengehäusebauteit 52 eine Durchbrechung 56 in einer Mantelfläche 54 aufweist, durch die hindurch der Betätigungstaster 70 in der durch den Pfeil 8 verdeutlichten Art und Weise eingedrückt werden kann, um hierdurch das Membranteil 60 in der durch den Pfeil 4 verdeutlichten Richtung zu verlagern.

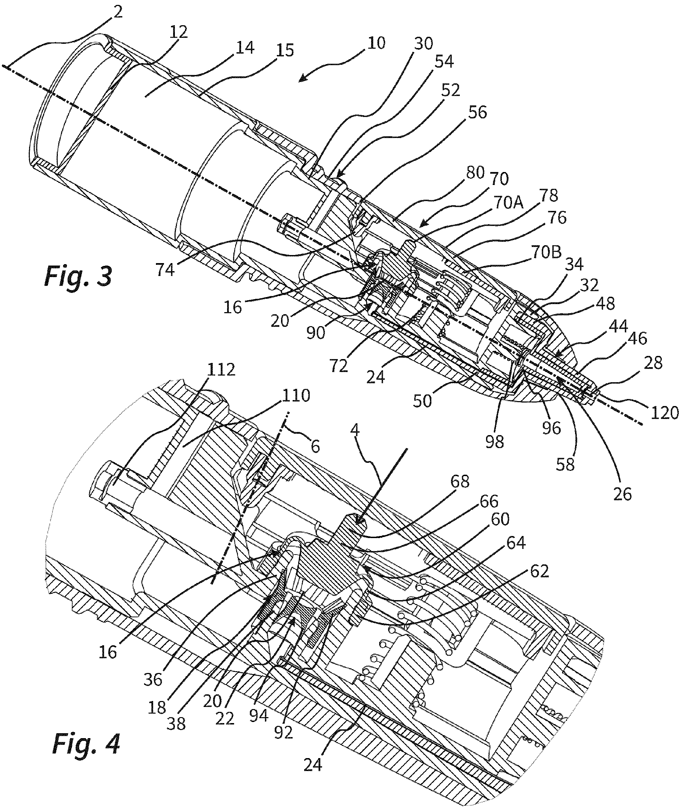

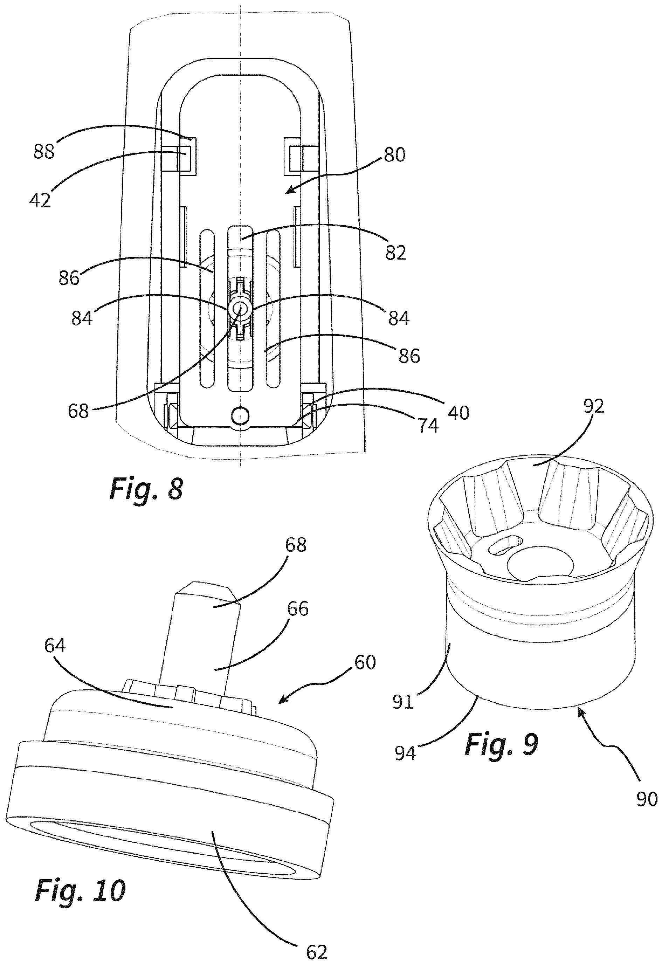

Anhand der perspektivischen Darstellungen der Fig. 3 und 4 sowie Bezug nehmend auf die Detaildarstellungen der Fig. 8 bis 10 wird der Spender weiter erläutert.

Vor dem Flüssigkeitsaustrag ist die Flüssigkeit im Flüssigkeitsspeicher 14 gelagert, welcher im Falle des Ausführungsbeispiels der Fig. 1A bis 10 durch eine Mantelfläche 15 begrenzt wird, die Teil des ersten Gehäusebauteils 30 ist, und der am proximalen Ende des Spenders durch einen eingerasteten Boden 12 verschlossen wird. Der Spender 10 wird üblicherweise in einer Ausrichtung verwendet, in der die Abgabeöffnung 28 nach unten weist, so dass die Flüssigkeit im Flüssigkeitsspeicher 14 während der Betätigung des Betätigungstasters 70 unmittelbar am Ventilbauteil 90 anliegt, nämlich an einer Einlassventillippe 92 des Einlassventils 18. Bei Unterdruck in der sich daran anschließenden Pumpkammer 20 kann die Flüssigkeit somit in die Pumpkammer 20 einströmen. Die Pumpkammer 20 selbst ist durch das genannte Ventilbauteil 90, einen ringförmigen Gehäuseabschnitt 36 des ersten Gehäusebauteils 30 sowie das bereits genannte in sich verformbare Membranteil 60 begrenzt.

Wenn das Membranteil in Richtung des Pfeils 4 und in Richtung des Ventilbauteils 90 verlagert wird, stellt sich ein Überdruck in der Pumpkammer ein, der ein Auslassventil 22 öffnet, indem dessen Auslassventillippen 94 ausgelenkt werden. Die Flüssigkeit kann hierdurch in einen Kanalabschnitt 24 strömen, der in Haupterstreckungsrichtung 2 ausgerichtet ist und bis zum distalen Ende des Gehäusebauteils 30 führt. An diesem distalen Ende des Gehäusebauteils 30 sind zwei ringförmige Stege 32, 34 vorgesehen. Der äußere Steg 32 weist einen Innendurchmesser auf, der an einen ringförmigen Steg 48 des zweiten Gehäusebauteils 44 derart angepasst ist, dass ein durch die beiden Gehäusebauteile 30, 44 definierter Zwischenraum gegenüber der Umgebung isoliert ist. An dem ringförmigen Steg 48 des zweiten Gehäusebauteils 44 ist ein lanzenförmiger Fortsatz vorgesehen, der einen Einsatz 50 bildet, welcher in den Kanalabschnitt 24 hineinragt und dessen Volumen zum überwiegenden Teil ausfüllt. Die Flüssigkeit, die durch den so bezüglich seines Volumens minimierten Kanalabschnitt 24 bis zum zweiten Gehäusebauteil 44 geströmt ist, kann hier nicht unmittelbar durch die Abgabeöffnung 28 austreten, da ein Abgabeventil 26 der Abgabeöffnung 28 vorgeschaltet ist, welches den bereits genannten Abgabeventilkörper 96 umfasst, der mittels der Abgabeventilfeder 98 abdichtend gegen die Abgabeöffnung 28 gedrückt wird und diese somit verschließt. Mit steigendem Flüssigkeitsdruck am Abgabeventil 26 wird jedoch der Abgabeventilkörper 96 gegen die Kraft der Abgabeventilfeder 98 verformt und öffnet somit den Flüssigkeitspfad, so dass die Flüssigkeit unter vergleichsweise geringem Druck durch die Abgabeöffnung 28 austreten kann und an den jenseits der Abgabeöffnung 28 vorgesehenen Tropfenbildungsmitteln in Form einer Tropfenbildungsfläche 120 einen Tropfen bildet. Erst wenn dieser ein durch die Geometrie derTropfenbildungsmittel definiertes Volumen aufweist, löst er sich und kann somit beispielsweise in ein Auge, ein Nasenloch oder ein Ohr des Benutzers appliziert werden.

Wenn der Betätigungstaster 70 nach erfolgter Betätigung vom Benutzer losgelassen wird, wird er von der in Art einer metallischen Schraubenfeder ausgestalteten Federeinrichtung 72 zurück in seine Ausgangslage gedrückt. Hierbei wird das Membranteil 60 mitgezogen und das Volumen der Pumpkammer 20 wieder vergrößert, so dass Flüssigkeit aus dem Flüssigkeitsspeicher 14 in die Pumpkammer 20 eingesogen wird. Um zu verhindern, dass hierdurch ein Unterdruck im Flüssigkeitsspeicher 14 entsteht, weist der Spender 10 einen Belüftungskanal 110 auf, der mit einem Sterilfilter 112 versehen ist und der ein Nachströmen von Luft aus einer Umgebung in den Flüssigkeitsspeicher 14 gestattet.

Sowohl das genannte Ventilbauteil 90 als auch das Membranteil 60 sind jeweils als Bauteil ausgestaltet, welches im Zweikomponentenspritzguss hergestellt wird. Beide Bauteile weisen jeweils einen starren Bauteilabschnitt auf, nämlich einen Befestigungsring 91, 62, an dem jeweils eine Komponente aus weichem Kunststoff angespritzt ist. Im Falle des in Fig. 9 vergrößert dargestellten Ventilbauteils bildet der weiche Kunststoff insbesondere die bereits genannten Komponenten der Einlassventillippe 92 und der aufgrund der Perspektive in Fig. 9 nicht erkennbaren Auslassventillippe 94. Im Falle des Membranteils 60 bilden die aus weichem Kunststoff hergestellten Bestandteile eine Pumpkammerwandung 64, die in Richtung der Pumpkammer 20 weist, und ein dickeres Mittelteil, welches auf der der Pumpkammer abgewandten Seite in einen Ausgleichsabschnitt 66 und einen Verbindungsabschnitt 68 übergeht.

Bezug nehmend wieder auf die geschnittenen Darstellungen der Fig. 4, in der das äußere Tasterbauteil 76 ausgeblendet ist, ist zu erkennen, dass der genannte Verbindungsabschnitt 68 in einem Verbindungsbereich 70A in das Tasterbauteil 80 hineinragt. Er ist hier mit einer Klemmverbindung gesichert. Diese Klemmverbindung ist anhand der Fig. 8 näher erläutert, welche einen Ausschnitt des Spenders bei abgenommenem Tasterbauteil 76 zeigt. Wie hier zu erkennen ist, sind in dem Tasterbauteil 80 drei Durchbrechungen 82, 86 vorgesehen. Die Durchbrechung 82 ist die eigentliche Kopplungsdurchbrechung. Sie ist in Form einer länglichen Durchbrechung gestaltet, deren einander gegenüberliegenden Längsseiten Klemmflächen 84 zur Festlegung des Verbindungsabschnitts bilden. Diese sind mit einer scharfkantigen Profilierung versehen, die sich von beiden Seiten in den Verbindungsabschnitt 68 hineindrücken und somit die gewünschte feste Klemmverbindung schaffen. Die hierzu parallel ausgerichteten Durchbrechungen 86 bilden Montagedurchbrechungen, die dem Zweck dienen, während der Montage eine Auslenkung der Stege zwischen der Kopplungsdurchbrechung und den Montagedurchbrechungen zu ermöglichen, ohne dass diese Stege oder andere Teile des Tasterbauteils 80 beschädigt werden.

Ebenfalls Bezug nehmend auf Fig. 8 wird die Befestigung des Tasterbauteils 80 erläutert. Das Tasterbauteil 80 verfügt über zwei angeformte Achsabschnitte 74, die die Kippachse 6 definieren. Diese Achsabschnitte 74 sind seitlich in Lagerösen 40 gelagert, die Teil des ersten Gehäusebauteils 30 sind. Am gegenüberliegenden Ende des Betätigungstasters 70 verfügt dieser über zwei ähnlich ausgestaltete Führungsösen 88, in die Nocken 42 des ersten Gehäusebauteils 30 hineinragen. Hierdurch wird verhindert, dass die Federeinrichtung 72 das Tasterbauteil 80 sowie den Kipptaster 70 als Ganzes aus dem Gehäuse herausdrückt.

Wie anhand der Fig. 3 und 4 gut zu ersehen ist, sind ein Verbindungsbereich 70A, in dem das Tasterbauteil 80 mit dem Verbindungsabschnitt 68 des Membranteils 60 verbunden ist, und ein Krafteinkopplungsbereich 70B, in dem die Federeinrichtung 72 auf das Tasterbauteil 76 wirkt, unterschiedlich weit von der Kippachse 6 beabstandet. Dies erleichtert zum einen die Montage der Einzelteile im begrenzten Bauraum. Zum anderen kann hierdurch die Auslegung des Spenders und die auf den Betätigungstaster 70 wirkende Rückstellkraft in vorteilhafter Weise angepasst werden.

Wie sich aus Fig. 8 ersehen lässt, ist das dort dargestellte innere Tasterbauteil 80 bezüglich seiner Formgebung vor allem im Hinblick auf seine technische Funktion ausgestaltet. Um dennoch eine ästhetisch ansprechende Gestaltung des Betätigungstasters 70 zu erzielen, ist das zweite, äußere Tasterbauteil 76 vorgesehen, welches mittels einer Rastverbindung mit dem inneren Tasterbauteil 80 verbunden ist. An diesem äußeren Tasterbauteil 76 ist die Druckfläche 78 vorgesehen, auf die ein Benutzer zum Zwecke der Betätigung seinen Daumen legt.

Wie bereits erläutert wurde, ist der dargestellte Spender als Tropfenspender ausgebildet. Dabei ist er derart ausgelegt, dass eine Betätigung des Betätigungstasters von einer unbetätigten Ausgangslage bis in seine betätigte Endlage den Austrag von genau einem Tropfen bewirken soll. Dementsprechend ist die Pumpkammer 20 recht klein ausgebildet und weist ein nutzbares Pumpkammervolumen, also eine Differenz zwischen Maximal- und Minimalvolumen der Pumpkammer 20, von 40µl auf.

Dieses sehr kleine Pumpvolumen führt dazu, dass besondere Vorkehrungen getroffen werden müssen, um den Spender in Betrieb zu nehmen. Im Lieferzustand des Spenders ist die Pumpkammer 20 mit Luft gefüllt. Ebenso ist der Flüssigkeitspfad von der Pumpkammer 20 bis zur Abgabeöffnung 28 mit Luft gefüllt.

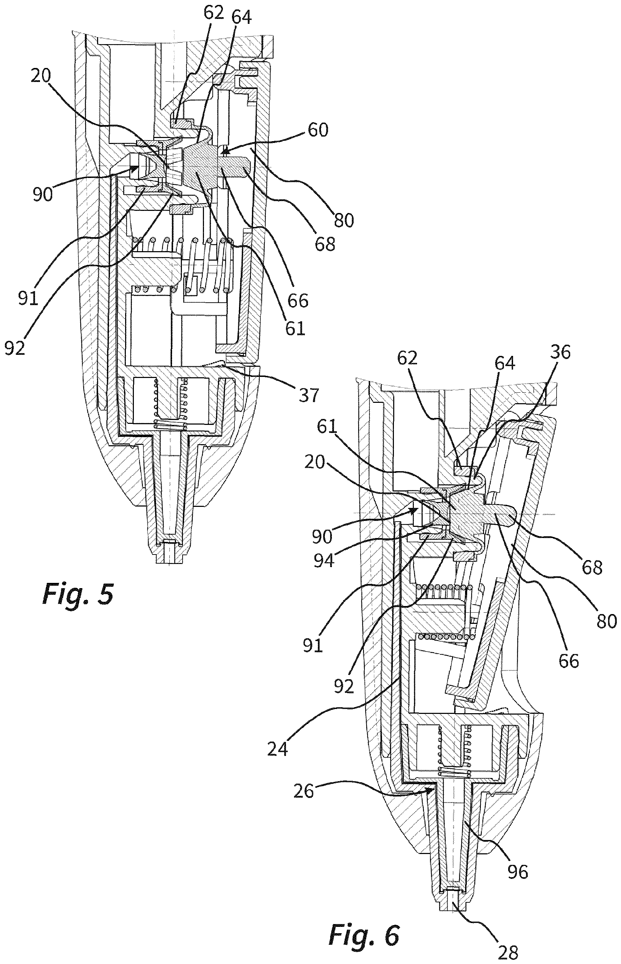

Damit es ausgehend von diesem Ausgangszustand überhaupt möglich ist, die Luft aus der Pumpkammer 20 zu verdrängen, weist die Pumpeinrichtung 16 einen sehr kleinen Quotienten zwischen dem Minimalvolumen der Pumpkammer 20 im betätigten Zustand und dem Maximalvolumen der Pumpkammer 20 im unbetätigten Zustand auf. Dies wird anhand der Fig. 5 und 6 dargestellt. Fig. 5 zeigt den unbetätigten Zustand. Fig. 6 zeigt den betätigten Zustand.

Es ist zu ersehen, dass im betätigten Zustand das Membranteil 60 derart weit in Richtung des auf einer ringförmigen Gegenwandung 38 aufgesetzten Ventilbauteils 90 verlagert ist, dass es mit diesem in Berührkontakt gelangt. Dort, wo das Membranteil 60 und das Ventilbauteil 90 in Berührkontakt gelangen, verbleibt kein Restvolumen zwischen ihnen. Es ist weiter zu ersehen, dass die Pumpkammerwandung 64, die durch das Membranteil 60 gebildet wird, sich bei der Überführung in den betätigten Zustand partiell umstülpt bzw. abrollt, so dass sie gegen Ende der Betätigung am ringförmigen Gehäuseabschnitt 36 anliegt oder nur noch ein sehr schmaler Spalt hierzwischen verbleibt. Das hierdurch erzielbare Minimalvolumen der Pumpkammer, welches in Fig. 6 dargestellt ist, beträgt weniger als 2 µl. Der Quotient aus Minimalvolumen und Maximalvolumen der Pumpkammer 20 liegt somit unter 1:20.

Zu erkennen ist anhand der Fig. 5 und 6 auch, dass die Kippbeweglichkeit des Betätigungstasters dazu führt, dass auch der Verbindungsabschnitt 68 des Membranteils 60 entsprechend verkippt wird. Um dennoch das genannte sehr geringe Minimalvolumen der Pumpkammer 20 zu ermöglichen, verformt sich der Ausgleichsabschnitt 66 in der in Fig. 6 dargestellten Weise.

Eine ähnliche Problematik wie bei der Pumpkammer 20 ist auch im Hinblick auf den Flüssigkeitspfad bis zur Abgabeöffnung 28 vorhanden. Auch hier ist es von Vorteil, wenn das Volumen möglichst gering ist, damit hier im Lieferzustand nur wenig Luft vorhanden ist, die vor dem Austrag ausgetrieben werden muss.

Hier ist es insbesondere die schon beschriebene Gestaltung des Einsatzes 50, die das Volumen stark reduziert, so dass die aus der Pumpkammer 20 in Richtung des Kanalabschnitts 24 ausgetriebene Luft nach zwei bis drei Betätigungsvorgängen des Betätigungstasters 70 einen ausreichenden Druck erreicht hat, um das Abgabeventil 26 zu öffnen.

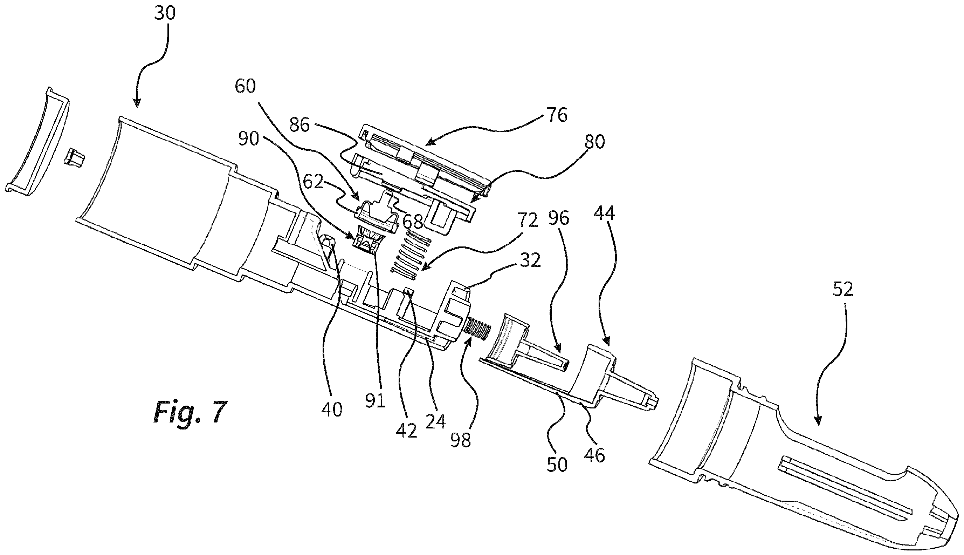

Anhand der Fig. 7 wird nachfolgend die Montagereihenfolge erläutert.

In den Fig. 5 und 6 ist gestrichelt auch eine optionale Raste 37 vorgesehen, die bei Betätigung eine Mindestkraft erzwingt. Dies ist insbesondere zweckmäßig, wenn der Spender für ein Pumpvolumen ausgebildet ist, welches geringer als jenes ist, das für eine rein schwerkraftbedingt Ablösung des Tropfens erforderlich ist. Bei derart geringen Volumina kann der durch die schlagartige Pumpkammerkomprimierung erzeugte Druckstoß dennoch ein Ablösen des Tropfens von der Tropfenbildungsfläche 120 bewirken.

Ausgehend von dem ersten Gehäusebauteil 30 wird zunächst das Ventilbauteil 90 eingefügt und mittels seines Befestigungsrings 91 an der ringförmigen Gegenwandung 38 des ersten Gehäusebauteils 30 befestigt. Anschließend werden die Rückstellfeder 72 und das Membranteil 60 eingefügt, wobei das Membranteil 60 mittels eines Befestigungsrings 62 an der Außenseite des ringförmigen Gehäuseabschnitts 36 festgeklemmt wird und hierbei eine flüssigkeitsdichte Verbindung schafft.

Anschließend erfolgt die Einfügung des inneren Tasterbauteils 80, welches hierbei im Bereich der Achsabschnitte 74 in die Lagerösen 40 hineingedrückt wird und im Bereich der Führungsösen 88 über die am ersten Gehäusebauteil 30 vorgesehenen Nocken 42 gedrückt wird. Während der Einfügung des Tasterbauteils 80 wird die Kopplungsdurchbrechung 82 von einem Spreizer geweitet, so dass der Verbindungsabschnitt 68 des Membranteils ohne Verformung in die Kopplungsdurchbrechung einfahren kann. Anschließend wird der Spreizer entfernt und die beschriebene Klemmverbindung so geschaffen.

Danach wird ein vormontierter Verbund von verformbarem Abgabeventilkörper 96 und zweitem Gehäusebauteil 44 stirnseitig auf das erste Gehäusebauteil 30 aufgeschoben, wobei zuvor die Abgabeventilfeder 98 an dieser Stirnseite aufgesetzt wurde und wobei weiterhin der Einsatz 50 in den Kanalabschnitt 24 des ersten Gehäusebauteils 30 eingefügt wird.

Als letzter Fertigungsschritt wird zunächst das Außengehäusebauteil 52 auf den Verbund der zuvor montierten Teile aufgeschoben, wobei hierdurch auch eine Sicherung des ersten Gehäusebauteils 30 und des zweiten Gehäusebauteils 44 aneinander erzielt wird. Anschließend wird das zweite Tasterbauteil 76 auf das erste Tasterbauteil 80 gedrückt und verrastet hier.

Die beiden letztgenannten Bauteile 52, 76 haben keinerlei Flüssigkeitskontakt und beeinflussen die Austragcharakteristik des Spenders daher nicht. Diese beiden Bauteile 52, 76 sind jene Bauteile, die bestimmungsgemäß im Hinblick auf Form und Farbe angepasst werden können, um den Spender 10 an individuelle Wünsche eines Herstellers von pharmazeutischen Flüssigkeiten anpassen zu können.

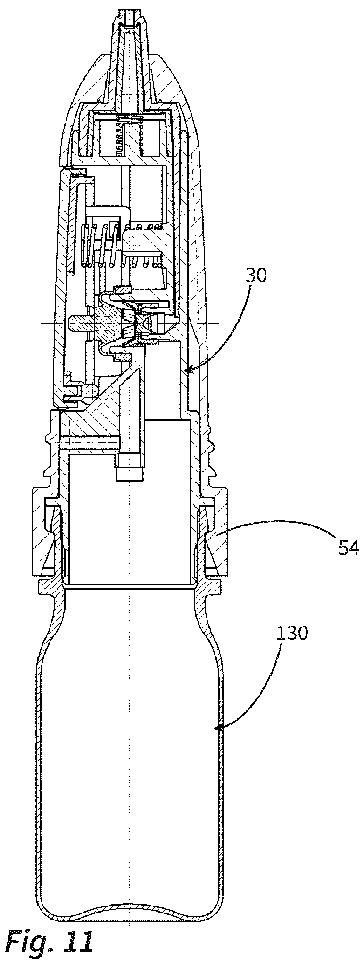

Fig. 11 zeigt ein zweites Ausführungsbeispiel eines erfindungsgemäßen Spenders. Dieser weist nur einen einzigen Unterschied zum vorgenannten Spender auf, nämlich eine Ausgestaltung des ersten Gehäusebauteils 30 und des Außengehäusebauteils 52 derart, dass diese die Ankopplung eines separaten Flaschenkörpers 130 gestatten.

## Patentansprüche

1. Spender (10) zum Austrag pharmazeutischer Flüssigkeiten, insbesondere für den tropfenweisen Austrag von pharmazeutischen Flüssigkeiten in Form von Einzeltropfen, mit den folgenden Merkmalen:
a. der Spender (10) weist einen Flüssigkeitsspeicher (14), eine Pumpeinrichtung (16) mit einer volumetrisch veränderlichen Pumpkammer (20) sowie eine Abgabeöffnung (28) zur Abgabe der Flüssigkeit auf, und
b. der Spender (10) weist ein die Pumpkammer (20) an einer Seite begrenzendes Membranteil (60) auf, das zumindest teilweise aus einem weichen Kunststoff besteht und das durch Verlagerung und/oder Verformung das Volumen der Pumpkammer (20) verändert, und
c. der Spender (10) weist ein Gehäuse, innerhalb dessen die Pumpeinrichtung vorgesehen ist, sowie einen gegenüber dem Gehäuse zwischen einer unbetätigten Ausgangslage und einer betätigten Endlage verlagerbaren Betätigungstaster (70) zur Verlagerung oder Verformung des Membranteils (60) und zur Verkleinerung der Pumpkammer (20) zum Zwecke des Flüssigkeitsaustrags auf, und
d. in einem äußeren Randbereich ist das Membranteil (60) umlaufend an einem Gehäuseabschnitt (36) des Gehäuses befestigt, und **gekennzeichnet durch** die folgenden weiteren Merkmale:
e. die Befestigung des Randbereichs des Membranteils (60) am Gehäuseabschnitt (36) erfolgt mittels eines Befestigungsrings (62) aus einem starren Kunststoff, wobei der Befestigungsring (62) gemeinsam mit Abschnitten des Membranteils (60) aus dem weichen Kunststoff einstückig ausgebildet ist.

2. Spender (10) nach Anspruch 1 mit dem folgenden weiteren Merkmal:
a. der Gehäuseabschnitt (36) ist ringförmig ausgebildet, wobei der Befestigungsring (62) an einer Außenseite des Gehäuseabschnitts (36) aufgeklemmt ist und wobei eine Innenseite des Gehäuseabschnitts (36) eine umlaufende Wandung der Pumpkammer (20) bildet.

3. Spender (10) nach einem der vorstehenden Ansprüche mit den folgenden Merkmalen:
a. das Gehäuse des Spenders (10) weist eine bezogen auf eine Haupterstreckungsrichtung (2) längliche Außenform auf, wobei am distalen Ende des Spenders (10) die Abgabeöffnung (28) vorgesehen ist, und
b. der Spender (10) weist den Betätigungstaster (70) auf, der im Bereich einer Mantelfläche (54) des Gehäuses seitlich am Spender (10) vorgesehen ist und in Richtung einer Mittelachse zur Verformung des Membranteils des Gehäuses eindrückbar ist.

4. Spender (10) nach einem der vorstehenden Ansprüche mit dem folgenden weiteren Merkmal:
a. der Spender (10) ist als Tropfenspender (10) ausgebildet und weist stromabwärts der Abgabeöffnung (28) Tropfenbildungsmittel auf,
vorzugsweise mit mindestens einem der folgenden zusätzlichen Merkmale:
b. die Tropfenbildungsmittel umfassen eine die Abgabeöffnung umgebende Tropfenbildungsfläche auf, welche vorzugsweise eben oder konkav geformt ist und/oder welche vorzugsweise von einer scharfkantigen Abrisskante umgeben sind, und/oder
c. der Spender weist eine Abstimmung zwischen der Pumpeinrichtung (16) und den Tropfenbildungsmitteln auf, durch die eine vollständige Betätigung des Betätigungstasters genau einen abfallenden Tropfen erzeugt.

5. Spender (10) nach einem der vorstehenden Ansprüche mit dem folgenden weiteren Merkmal:
a. der Spender (10) weist eine Spenderkappe (100) auf, die auf das Gehäuse aufsetzbar ist und im aufgesetzten Zustand die Abgabeöffnung schützt,
vorzugweise mit mindestens einem der folgenden zusätzlichen Merkmale:
b. die Spenderkappe (100) ist als belüftete Spenderkappe ausgestaltet und weist eine Ventilationsöffnung (102) auf, durch die die Abgabeöffnung (28) auch bei aufgesetzter Spenderkappe (100) mit einer umgebenden Atmosphäre verbunden oder verbindbar ist, wobei der Ventilationsöffnung (102) vorzugsweise ein Sterilfilter (104) zugeordnet ist, und/oder
c. die Spenderkappe (100) weist ein Pad (106) auf, welches bei aufgesetzter Spenderkappe (100) derart über oder an der Abgabeöffnung (28) positioniert ist, dass ein stromabwärts der Abgabeöffnung (28) verbleibender Flüssigkeitsrest hiervon aufgenommen und/oder dekontaminiert wird.

6. Spender (10) nach einem der vorstehenden Ansprüche mit mindestens einem der folgenden Merkmale:
a. die Differenz zwischen einem maximalen Pumpkammervolumen und einem minimalen Pumpkammervolumen beträgt zwischen 20 µl und 50 µl, vorzugsweise zwischen 30 µl und 40 µl, und/oder
b. der Quotient zwischen einem minimalen Pumpkammervolumen und einem maximalen Pumpkammervolumen beträgt maximal 1:2, vorzugsweise maximal 1:3, und/oder
c. der Spender (10) weist stromaufwärts der Abgabeöffnung (28) ein Abgabeventil (26) auf, wobei das Abgabeventil vorzugsweise zum Öffnen ab einem Überdruck von 0,3 bar ausgebildet ist, und/oder
d. der Flüssigkeitsspeicher (14) des Spenders weist eine Mantelfläche (15) auf, die einstückig mit dem die Pumpeinrichtung (16) umgebenden Gehäuse ausgebildet ist, wobei vorzugsweise an einem der Abgabeöffnung (28) gegenüberliegenden Ende ein als separates Teil ausgebildeter Boden (12) an der Mantelfläche (15) befestigt ist und/oder
e. der Flüssigkeitsspeicher ist mittels eines Belüftungskanals mit einer umgebenden Atmosphäre verbunden oder der Flüssigkeitsspeicher weist ein variables Innenvolumen auf und ist hierfür durch eine flexible oder verlagerbare Wandung begrenzt.

## Claims

1. Dispenser (10) for discharge of pharmaceutical liquids, especially for the dropwise discharge of pharmaceutical liquids in the form of individual droplets, having the following features:
a. the dispenser (10) has a liquid reservoir (14), a pump device (16) having a volumetrically variable pump chamber (20), and a delivery opening (28) for delivery of the liquid, and
b. the dispenser (10) has a membrane portion (60) that bounds the pump chamber (20) on one side, which consists at least partly of a soft plastic and which alters the volume of the pump chamber (20) by displacement and/or deformation, and
c. the dispenser (10) has a housing within which the pump device is provided, and an actuating button (70), displaceable relative to the housing between an unactuated starting position and an actuated final position, for displacing or deforming the membrane portion (60) and for reducing the size of the pump chamber (20) for the purpose of liquid discharge, and
d. the membrane portion (60) is secured circumferentially to a housing section (36) of the housing in an outer edge region, and **characterized by** the following further features:
e. the edge region of the membrane portion (60) is secured to the housing section (36) by means of a securing ring (62) made of a rigid plastic, wherein the securing ring (62) is formed in one piece from the soft plastic together with sections of the membrane portion (60).

2. Dispenser (10) according to Claim 1, having the following further feature:
a. the housing section (36) is in annular form, wherein the securing ring (62) is clamped to an outer face of the housing section (36) and wherein an inner face of the housing section (36) forms a circumferential wall of the pump chamber (20).

3. Dispenser (10) according to either of the preceding claims, having the following features:
a. the housing of the dispenser (10) has an elongate outer shape based on a main direction of extension (2), wherein the delivery opening (28) is provided at the distal end of the dispenser (10), and
b. the dispenser (10) has the actuating button (70) which is provided in the region of an outer surface (54) of the housing at the side of the dispenser (10) and can be depressed in the direction of a center axis for deformation of the membrane portion of the housing.

4. Dispenser (10) according to any of the preceding claims, having the following further feature:
a. the dispenser (10) is designed as a droplet dispenser (10) and has means of droplet formation downstream of the delivery opening (28),
preferably having at least one of the following additional features:
b. the means of droplet formation comprise a droplet formation surface which surrounds the delivery opening and is preferably in flat or concave form and/or which is preferably surrounded by a sharp break-off edge, and/or
c. the dispenser has a coordination between the pump device (16) and the means of droplet formation such that a complete actuation of the actuating button produces exactly one falling droplet.

5. Dispenser (10) according to any of the preceding claims, having the following further feature:
a. the dispenser (10) has a dispenser cap (100) that can be placed onto the housing and protects the delivery opening when in place,
preferably having at least one of the following additional features:
b. the dispenser cap (100) is configured as a ventilated dispenser cap and has a ventilation opening (102) through which the delivery opening (28) is or can be connected to a surrounding atmosphere even with the dispenser cap (100) in place, preferably with a sterile filter (104) being assigned to the ventilation opening (102), and/or
c. the dispenser cap (100) has a pad (106) which, with the dispenser cap (100) in place, is positioned over or on the delivery opening (28) such that any liquid residue that remains downstream of the delivery opening (28) is absorbed and/or decontaminated thereby.

6. Dispenser (10) according to any of the preceding claims, having at least one of the following features:
a. the difference between a maximum pump chamber volume and a minimum pump chamber volume is between 20 µl and 50 µl, preferably between 30 µl and 40 µl, and/or
b. the quotient between a minimum pump chamber volume and a maximum pump chamber volume is not more than 1:2, preferably not more than 1:3, and/or
c. the dispenser (10) has a delivery valve (26) upstream of the delivery opening (28), wherein the delivery valve is preferably designed to open over and above a positive pressure of 0.3 bar, and/or
d. the liquid reservoir (14) of the dispenser has an outer surface (15) in one-piece form together with the housing surrounding the pump device (16), wherein a base (12) in the form of a separate part is preferably secured to the outer surface (15) at an opposite end from the delivery opening (28), and/or
e. the liquid reservoir is connected to a surrounding atmosphere by means of a ventilation channel, or the liquid reservoir has a variable internal volume and is bounded for the purpose by a flexible or displaceable wall.

## Revendications

1. Distributeur (10) pour le déchargement de liquides pharmaceutiques, notamment pour le déchargement goutte à goutte de liquides pharmaceutiques sous forme de gouttes individuelles, présentant les caractéristiques suivantes :
a. le distributeur (10) présente un réservoir de liquide (14), un dispositif de pompage (16) comprenant une chambre de pompage (20) variable en volume ainsi qu'une ouverture de distribution (28) pour la distribution du liquide, et
b. le distributeur (10) présente une partie membrane (60) délimitant la chambre de pompage (20) sur un côté, qui est constituée au moins partiellement d'une matière plastique souple et qui modifie le volume de la chambre de pompage (20) par déplacement et/ou déformation, et
c. le distributeur (10) présente un boîtier, à l'intérieur duquel est prévu le dispositif de pompage, ainsi qu'un bouton-poussoir d'actionnement (70) déplaçable par rapport au boîtier entre une position initiale non activée et une position finale activée, pour déplacer ou déformer la partie membrane (60) et pour réduire la taille de la chambre de pompage (20) afin de décharger le liquide, et
d. dans une zone de bord extérieure, la partie membrane (60) est fixée de manière périphérique à une section de boîtier (36) du boîtier, et **caractérisé par** les caractéristiques supplémentaires suivantes :
e. la fixation de la zone de bord de la partie membrane (60) sur la section de boîtier (36) s'effectue au moyen d'une bague de fixation (62) en une matière plastique rigide, la bague de fixation (62) étant réalisée d'un seul tenant conjointement avec des sections de la partie membrane (60) en la matière plastique souple.

2. Distributeur (10) selon la revendication 1, présentant la caractéristique supplémentaire suivante :
a. la section de boîtier (36) est réalisée sous forme annulaire, la bague de fixation (62) étant serrée sur un côté extérieur de la section de boîtier (36), et un côté intérieur de la section de boîtier (36) formant une paroi périphérique de la chambre de pompage (20).

3. Distributeur (10) selon l'une quelconque des revendications précédentes, présentant les caractéristiques suivantes :
a. le boîtier du distributeur (10) présente une forme extérieure allongée par rapport à une direction d'extension principale (2), l'ouverture de distribution (28) étant prévue à l'extrémité distale du distributeur (10), et
b. le distributeur (10) présente le bouton-poussoir d'actionnement (70), qui est prévu dans la zone d'une surface d'enveloppe (54) du boîtier, latéralement sur le distributeur (10), et qui peut être enfoncé dans la direction d'un axe médian pour déformer la partie membrane du boîtier.

4. Distributeur (10) selon l'une quelconque des revendications précédentes, présentant la caractéristique supplémentaire suivante :
a. le distributeur (10) est réalisé sous forme de distributeur de gouttes (10) et présente en aval de l'ouverture de distribution (28) des moyens de formation de gouttes,
de préférence présentant au moins l'une des caractéristiques supplémentaires suivantes :
b. les moyens de formation de gouttes comprennent une surface de formation de gouttes entourant l'ouverture de distribution, qui est de préférence de forme plane ou concave et/ou qui est de préférence entourée par un bord de rupture à arête vive, et/ou
c. le distributeur présente une coordination entre le dispositif de pompage (16) et les moyens de formation de gouttes, par laquelle un actionnement complet du bouton-poussoir d'actionnement produit exactement une goutte descendante.

5. Distributeur (10) selon l'une quelconque des revendications précédentes, présentant la caractéristique supplémentaire suivante :
a. le distributeur (10) présente un capuchon de distributeur (100), qui peut être monté sur le boîtier et qui, à l'état monté, protège l'ouverture de distribution,
de préférence présentant au moins l'une des caractéristiques supplémentaires suivantes :
b. le capuchon de distributeur (100) est conçu sous forme de capuchon de distributeur aéré et présente une ouverture de ventilation (102), par laquelle l'ouverture de distribution (28) est ou peut être reliée à une atmosphère environnante même lorsque le capuchon de distributeur (100) est monté, un filtre stérile (104) étant de préférence associé à l'ouverture de ventilation (102), et/ou
c. le capuchon de distributeur (100) présente un tampon (106) qui, lorsque le capuchon de distributeur (100) est monté, est positionné au-dessus ou sur l'ouverture de distribution (28) de telle sorte qu'un résidu de liquide restant en aval de l'ouverture de distribution (28) est absorbé et/ou décontaminé par celui-ci.

6. Distributeur (10) selon l'une quelconque des revendications précédentes, présentant au moins l'une des caractéristiques suivantes :
a. la différence entre un volume de chambre de pompage maximal et un volume de chambre de pompage minimal est comprise entre 20 µl et 50 µl, de préférence entre 30 µl et 40 µl, et/ou
b. le quotient entre un volume de chambre de pompage minimal et un volume de chambre de pompage maximal est de 1:2 au maximum, de préférence de 1:3 au maximum, et/ou
c. le distributeur (10) présente une soupape de distribution (26) en amont de l'ouverture de distribution (28), la soupape de distribution étant de préférence réalisée pour s'ouvrir à partir d'une surpression de 0,3 bar, et/ou
d. le réservoir de liquide (14) du distributeur présente une surface d'enveloppe (15), qui est réalisée d'un seul tenant avec le boîtier entourant le dispositif de pompage (16), un fond (12) réalisé en tant que partie séparée étant de préférence fixé sur la surface d'enveloppe (15) à une extrémité opposée à l'ouverture de distribution (28) et/ou
e. le réservoir de liquide est relié à une atmosphère environnante au moyen d'un canal d'aération ou le réservoir de liquide présente un volume intérieur variable et est délimité à cet effet par une paroi flexible ou déplaçable.
